(19) Europäisches Patentamt
European Patent Office
Office européen des brevets

(11) Veröffentlichungsnummer: **0 226 132**
**A2**

(12) # EUROPÄISCHE PATENTANMELDUNG

(21) Anmeldenummer: **86116833.4**

(22) Anmeldetag: **03.12.86**

(51) Int. Cl.[4]: **C 07 K 5/02**
C 07 K 5/06, A 01 N 57/20
A 01 N 57/22

(30) Priorität: **14.12.85 DE 3544376**

(43) Veröffentlichungstag der Anmeldung:
**24.06.87 Patentblatt 87/26**

(84) Benannte Vertragsstaaten:
**AT BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Kehne, Heinz, Dr.**
**Berliner Strasse 10**
**D-6238 Hofheim am Taunus(DE)**

(72) Erfinder: **Bauer, Klaus, Dr.**
**Kolpingstrasse 7**
**D-6054 Rodgau(DE)**

(72) Erfinder: **Bieringer, Hermann, Dr.**
**Eichenweg 26**
**D-6239 Eppstein/Taunus(DE)**

(54) **Dipeptide mit C-terminalem Phosphinothricin, Verfahren zu ihrer Herstellung und ihre Verwendung zur Bekämpfung unerwünschten Pflanzenwuchses.**

(57) Dipeptide mit C-terminalem Phosphinothricin der Formel

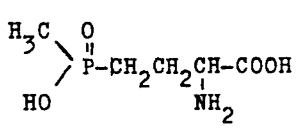

in denen Phosphinothricin an der Aminogruppe mit einer - vorzugsweise natürlichen - Aminosäure verknüpft ist, sind wirksame Herbizide im Vor- und Nachauflauf. Man erhält die Verbindungen z.B. durch Umsetzung von Phosphinothricin mit dem Hydroxysuccinimidester einer an der Aminogruppe geschützten Aminosäure in Gegenwart einer Base und anschließende Abspaltung der Schutzgruppen.

EP 0 226 132 A2

Dipeptide mit C-terminalem Phosphinothricin, Verfahren zu ihrer Herstellung und ihre Verwendung zur Bekämpfung unerwünschten Pflanzenwuchses

Es ist bekannt, daß Phosphinothricin (Ptc)

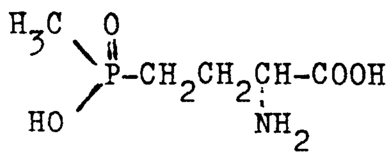

und einige Peptide mit N-terminalem Ptc eine gute herbizide Wirksamkeit aufweisen (DE-PS 27 17 440; DE-PS 28 48 224). Es wurde nun gefunden, daß auch Peptide mit C-terminalem Ptc eine ausgezeichnete Wirkung als Herbizide besitzen, die derjenigen des Ptc gleichkommt und sie teilweise noch übertrifft.

Gegenstand der Erfindung sind daher Dipeptide der allgemeinen Formel I,

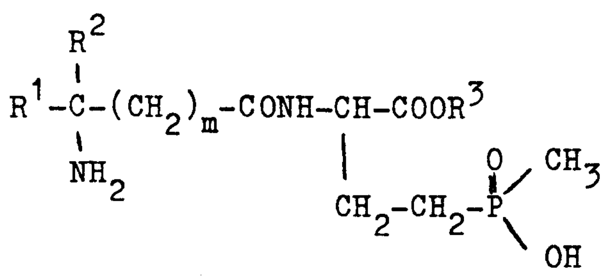

(I)

worin

$R^1$ und $R^2$ unabhängig voneinander Wasserstoff, $(C_1-C_{12})$-Alkyl, $(C_3-C_8)$Cycloalkyl, Phenyl, Benzyl oder Phenethyl, wobei die genannten Gruppen ihrerseits durch Halogen, $NH_2$, OH, SH, $NO_2$, $(C_1-C_4)$-Alkoxy, $(C_1-C_4)$Alkylthio, $CF_3$, $(C_1-C_4)$Alkoxycarbonyl, COOH oder $CONH_2$ substituiert sein können, oder

$R^1$ und $R^2$ gemeinsam eine Alkylenkette von 2 - 7 C-Atomen,

$R^3$ Wasserstoff oder $(C_1-C_{12})$-Alkyl und

m 0, 1, 2 oder 3

bedeuten, sowie deren Salze mit Basen und Säuren.

Die Verbindungen der Formel I enthalten 1 oder 2 asymmetrische C-Atome. Sie können also in mehreren stereoisomeren Formen auftreten. Die Erfindung betrifft daher nicht nur die Racemate, sondern auch deren Stereoisomere und Gemische derselben in beliebigen Verhältnissen.

Bevorzugte Verbindungen sind solche, in denen

$R^1$ und $R^2$ unabhängig voneinander Wasserstoff, $(C_1-C_8)$-Alkyl, Phenyl, Benzyl und Phenylethyl, wobei die genannten Gruppen ihrerseits durch $NH_2$, OH, SH, $(C_1-C_4)$Alkoxy, $(C_1-C_4)$Alkylthio, $(C_1-C_4)$-Alkoxycarbonyl, COOH oder $CONH_2$ substituiert sein können,

$R^1$ und $R^2$ zusammen mit dem sie verbindenden C-Atom einen Cyclopentyl- oder Cyclohexylrest,

$R^3$ Wasserstoff oder Methyl und m O, 1 oder 2 bedeuten, sowie deren Salze.

Besonders bevorzugt sind Dipeptide des Ptc mit "natürlichen", d.h. proteinogenen Aminosäuren wie Alanin (Ala), α-Aminobuttersäure (Abu), Asparagin (Asn), Asparaginsäure (Asp), Cystein (Cys), Glutamin (Gln), Glutaminsäure (Glu), Glycin (Gly), Isoleucin (Ile), Leucin (Leu), Lysin (Lys), Methionin (Met), Ornithin (Orn), Phenylalanin (Phe), Serin (Ser), Threonin (Thr), Tyrosin (Tyr), Valin (Val).

Auch nicht-proteinogene Aminosäuren kommen als Dipeptid-Komponente in Betracht, z.B. Phenylglycin (Phg), Norvalin (Nva).

Da die Verbindungen der Formel I saure (-COOH, -P(O)OH) und basische ($-NH_2$) Gruppen enthalten, sind sie zur Salzbildung mit Basen und starken Säuren befähigt. Als Salze mit Basen kommen insbesondere die Alkali- und Erdalkalisalze (mit $Na^+$, $K^+$ oder $Ca^{++}$), die Ammoniumsalze sowie Salze mit organischen Ammoniumbasen wie Mono-, Di- und Tri$(C_1-C_4)$alkyl- bzw. -hydroxyethylammoniumsalze, ferner die Benzyl-, Dibenzyl-, Phenyl-, Diphenyl- und Dicyclohexylammoniumsalze in Betracht. Zur Salzbildung an

der Aminogruppe eignen sich besonders starke Säuren wie HCl, HBr, $H_2SO_4$, $HNO_3$, $H_3PO_4$, $HClO_4$ und Benzolsulfonsäure.

Gegenstand der Erfindungen ist auch ein Verfahren zur Herstellung der Verbindungen der allgemeinen Formel I, das dadurch gekennzeichnet ist, daß man

a) eine Verbindung der Formel II

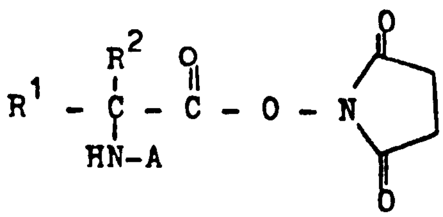

(II)

worin A eine Amino-Schutzgruppe bedeutet, mit Phosphinothricin in Gegenwart einer Hilfsbase oder

b) eine Verbindung der Formel III

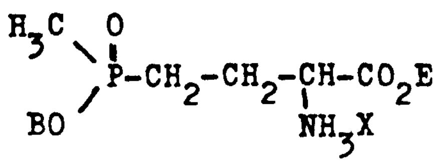

(III)

worin B und E Säureschutzgruppen sind und X das Anionäquivalent einer starken Säure bedeutet, mit einer Verbindung der Formel IV

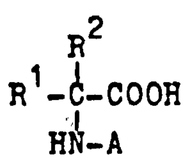

(IV)

in Gegenwart einer Hilfsbase und eines Kondensationsmittels

umsetzt und anschließend, soweit gewünscht, die vorhandenen Schutzgruppen in bekannter Weise abspaltet.

Die für die Verfahrensvariante a) benötigten N-Acyl-aminosäure-(N-hydroxysuccinimid)-ester der Formel II sind nach literaturbekannten Methoden darstellbar (J. Am. Chem. Soc. 86, 1839 (1964), Houben-Weyl Bd. 15/2, S. 149 ff). Zu ihrer Herstellung kommen als Ausgangsstoffe vor allem die natürlichen Aminosäuren wegen ihrer leichten Zugänglichkeit in Betracht. Als Schutzgruppen A verwendet man die in der Peptidchemie üblichen, z.B. die Carbobenzoxy-(Cbo-) und die tert. Butyloxycarbonyl-(Boc-)gruppe. Die Umsetzung von II mit Phosphinothricin führt man zweckmäßigerweise in einem organisch-wäßrigen Lösungsmittelgemisch wie Ethanol/Wasser, 1,4-Dioxan/Wasser, Tetrahydrofuran/Wasser, 1,2-Dimethoxyethan/Wasser oder in einem polaren, organischen Lösungsmittel wie Dimethylformamid bei Temperaturen zwischen -20°C und 60°C, vorzugsweise zwischen -10°C und 40°C aus. Als Hilfsbasen eignen sich in organisch wäßrigen Systemen besonders Alkalimetallhydroxide wie Natriumhydroxid, in polaren organischen Systemen besonders tertiäre Amine wie Triethylamin oder N-Ethylmorpholin.

Die für die Verfahrensvariante b) benötigten Verbindungen der Formeln III und IV erhält man nach allgemein bekannten Methoden (DOS 2717440; Houben-Weyl Bd. 15/1, S. 315, Bd. 12/1, S. 247 f. u. 423, Bd. 15/1, S. 46 ff). Auch hier schützt man die funktionellen Gruppen in den Ausgangsstoffen III und IV nach bekannten Methoden der Peptidchemie; z.B. eignen sich zum Schutz der Säurefunktionen in III Niederalkyl- und Benzylgruppen. Die Knüpfung der Peptidbindung erfolgt in einem inerten organ. Lösungsmittel wie $CH_2Cl_2$, Tetrahydrofuran, 1,4-Dioxan, Acetonitril, Dimethylformamid, Essigsäureethylester oder Dimethoxyethan bei Temperaturen zwischen -20°C und 80°C, bevorzugt zwischen -10°C und 50°C, in Gegenwart eines Kondensationsmittels und einer Hilfsbase. Als Kondensationsmittel können Chlorameisensäureester wie Chlorameisensäureethylester oder Dialkyl-

carbodiimide wie Dicyclohexylcarbodiimid dienen. Geeignete Hilfsbasen sind tertiäre organische Amine wie Triethylamin, N-Ethylmorpholin oder Pyridin.

Bei den Verfahrensvarianten a) und b) verbleiben nach der Peptidverknüpfung Schutzgruppen im Molekül, die nach literaturbekannten Methoden abgespalten werden können, z.B. die Cbo-Gruppe durch Hydrogenolyse, die Boc-Gruppe durch Acidolyse, Estergruppen durch saure oder alkalische Hydrolyse.

Je nach Art und Reihenfolge der Schutzgruppenabspaltung erhält man die erfindungsgemäßen Verbindungen I in freier Form oder als Salze. Die Salze können nach literaturbekannten Methoden (z.B. durch Umsetzung der Hydrochloride mit Propylenoxid) in die freien Dipeptide übergeführt werden. Aus diesen erhält man Salze durch Titration mit einem Äquivalent Säure bzw. Base und Eindampfen der wäßrigen Lösung.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Die Verbindungen der Formel I können mit zahlreichen anderen Herbiziden kombiniert werden; die Mischungen besitzen teilweise synergistische Wirkung. Beispiele für solche Mischungspartner sind:

Triazine wie 2-Chlor-4-ethylamino-6-isopropylamino-1,3,5-triazin (Atrazin), 2-Chlor-4,6-bis(ethylamino)-1,3,5-triazin (Simazin), 2-tert.-Butylamino-4-chlor-6-ethylamino-1,3,5-triazin (Terbutylazine), 2-tert.-Butylamino-4-ethylamino-6-methoxy-1,3,5-triazin (Terbumeton), 2-Ethylamino-4-isopropylamino-6-methylthio-1,3,5-triazin (Ametryne), 2,4-Bis(isopropylamino)-6-methylthio-1,3,5-triazin (Prometryn) oder 2-tert.-Butylamino-4-ethylamino-6-methylthio-1,3,5-triazin (Terbutryne),

Wuchsstoffe wie 2,4-Dichlorphenoxyessigsäure (2,4-D), 4-Chlor-2-methylphenoxyessigsäure (MCPA), 4-Chlor-2-methylphenoxypropionsäure (CMPP), 2-(2,4-Dichlorphenoxy)-propionsäure (2,4-DP), 2,4,5-Trichlorphenoxyessigsäure (2,4,5-T), 3,6-Dichlor-2-methoxybenzoesäure (Dicamba) und deren Ester und Salze,

Acetanilide wie 2-Chlor-2',6'-diethyl-N-methoxymethylacetanilid Alachlor, 2-Chlor-6'-ethyl-N-(2-methoxy-1-methylethyl)-acet-o-toluidid Metolachlor oder 2-Chlor-N-(ethoxymethyl)-6'-ethylacet-o-toluidid,

Phenylharnstoffe wie 3-(3,4-Dichlorphenyl)-1-methoxy-1-methylharnstoff (Linuron), 3-(3,4-Dichlorphenyl)-1,1-dimethylharnstoff (Diuron), 3-(4-Chlorphenyl)-1-methoxy-1-methylharnstoff (Monolinuron),

ferner 2-[3-(4,6-Dimethylpyrimidin-2-yl)ureidosulfonyl]-benzoesäuremethylester, 2-(4-Isopropyl-4-methyl-5-oxo-2-imidazolin-2-yl)-nicotinsäure-isopropylammoniumsalz, 2-(4-Isopropyl)-4-methyl-5-oxo-2-imidazolin-2-yl)chinolin-3-carbonsäure, 2-[3-(4-Chlor-6-methoxypyrimidin-2-yl)-ureidosulfonyl]-benzoesäuremethylester, 4-Amino-6-tert.-butyl-4,5-dihydro-3-methylthio-1,2,4-triazin-5-on, 3-Amino-1H-1,2,4-triazol, 2,6-Dinitro-N,N-dipropyl-4-trifluormethylanilin (Trifluralin), Ammoniumethylcarbamoylphosphonat und N-(Phosphonomethyl)glycin (Glyphosate).

Bei Applikation der Wirkstoffe auf die grünen Pflanzenteile im Nachauflaufverfahren tritt sehr rasch nach der Behandlung ein drastischer Wachstumsstop ein, und die Unkrautpflanzen bleiben in dem zum Applikationszeitpunkt vorhandenen Wachstumsstadium stehen und sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Die erfindungsgemäßen Verbindungen besitzen auch eine ausgezeichnete und sehr breite Wirkung gegen ein weites Spektrum annueller sowie perennierender Ungräser und Unkräuter, die an Wegrändern, in Industrie- oder Eisenbahnanlagen wachsen. Die Wirkstoffe eignen sich deshalb sowohl zur Anwendung auf nicht landwirtschaftlich genutzten Flächen als auch zur Unkrautbekämpfung in der Landwirtschaft. Der Einsatz von nichtselektiven Verbindungen in ein oder mehrjährigen landwirtschaftlichen Kulturen ist möglich, sofern durch die Art der Applikation und/oder das Alter der Kulturpflanzen sichergestellt wird, daß die Kulturpflanzen bzw. ihre empfindlichen grünen Teile nicht besprüht werden und so keinen Schaden leiden. Beispiele für derartige Einsatzmöglichkeiten sind Plantagen, Baumkulturen, Rebanlagen etc.

Da eine Anwendung der neuen Verbindungen den einjährigen Nutzkulturen vor dem Auflaufen der Kulturpflanzen oder in deren Reifestadium keinen Schaden zufügt, kann man sie auch vor der Aussaat, kurz vor oder nach der Ernte im Zuge der minimalen Bodenbearbeitung, der Bekämpfung von Spätverunkrautung und der Ernteerleichterung einsetzen.

Gegenstand der Erfindung sind daher auch herbizide Mittel, die die Verbindung der Formel I enthalten, und deren Verwendung zur Bekämpfung von unerwünschtem Pflanzenwuchs.

./.

Die erfindungsgemäßen Mittel enthalten die Wirkstoffe gemäß der allgemeinen Formel I zu 2-95%. Da die Wirkstoffe zum Teil wasserlöslich sind, können sie vorteilhaft als wäßrige Lösungen eingesetzt werden. Andernfalls können sie als emulgierbare Konzentrate, benetzbare Pulver und versprühbare Lösungen in den üblichen Zubereitungsformen angewendet werden, sofern sie nicht selbst wasserlöslich sind.

Benetzbare Pulver sind in Wasser gleichmäßig dispergierbare Präparate, die neben dem Wirkstoff außer einem Verdünnungs- oder Inertstoff noch Netzmittel, z.B. polyoxäthylierte Alkylphenole, polyoxäthylierte Oleyl- oder Stearylamine, Alkyl- oder Alkylphenyl-sulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Xylol oder auch höhersiedenden Aromaten erhalten.

Um in Wasser gute Suspensionen oder Emulsionen zu erreichen, werden weiterhin Netzmittel aus der obengenannten Reihe zugesetzt.

Bei herbiziden Mitteln können die Konzentrationen der Wirkstoffe in den handelsüblichen Formulierungen verschieden sein. In benetzbaren Pulvern variiert die Wirkstoffkonzentration z. B. zwischen etwa 10% und 80%, der Rest besteht aus den oben angegebenen Formulierungszusätzen. Bei emulgierbaren Konzentraten ist die Wirkstoffkonzentration etwa 10% bis 60%.

Zur Anwendung werden die handelsüblichen Konzentrate gegebenenfalls in üblicher Weise verdünnt, z. B. bei benetzbaren Pulvern und emulgierbaren Konzentraten mittels Wasser,

./.

versprühbare Lösungen werden vor der Anwendung nicht mehr mit weiteren inerten Stoffen verdünnt. Mit den äußeren Bedingungen wie Temperatur, Feuchtigkeit u.a. variiert die erforderliche Aufwandmenge. Sie kann in weiten Grenzen schwanken, z. B. zwischen 0,1 kg/ha und 10 kg/ha Aktivsubstanz, liegt jedoch vorzugsweise zwischen 0,3 und 3 kg/ha.

Folgende Beispiele dienen der weiteren Erläuterung der Erfindung

## A. Chemische Beispiele

### Beispiel 1: N-DL-Valyl-DL-phosphinothricin

36,2 g (0,2 mol) DL-Phosphinothricin werden in 200 ml 2 N NaOH gelöst. Bei 0°C tropft man 34,8 g (0,1 mol) Cbo-DL-Valin-hydroxysuccinimidester gelöst in 60 ml Dioxan zu. Man läßt auf Raumtemperatur kommen und gibt bis zur vollständigen Lösung Dioxan zu (ca. 250 ml). Nach 15 stündigem Nachrühren bei Raumtemperatur entfernt man das Dioxan im Vakuum, wäscht die Wasserphase mit Ether und säuert mit HCl bis pH 1 an. Extraktion der Wasserphase mit Essigester, Trocknen über $Na_2SO_4$ und Eindampfen liefert 39,2 g rohes N-Cbo-DL-Valyl-DL-phosphinothricin.

Die Abspaltung der Cbo-Schutzgruppe erfolgt durch Lösen in 200 ml Methanol, Zugeben von 4 g Palladium auf Aktivkohle (5 %) und Einleiten eines $H_2$-Stromes in die auf 40°C erwärmte Lösung bis keine $CO_2$-Entwicklung mehr zu beobachten ist. Man filtriert den Katalysator ab und entfernt das Lösungsmittel im Vakuum. Es hinterbleiben 15,3 g (Ausbeute über beide Stufen 55%) N-DL-Valyl-DL-phosphinothricin vom Schmp. 128-132°C (Zersetzung).

### Beispiel 2: N-L-Phenylalanyl-DL-phosphinothricin

13,2 g (0,036 mol) DL-Phosphinothricindimethylesterhydrobenzolsulfonat und 10,8 g (0,036 mol) Cbo-L-Phenylalanin werden mit 3,6 g (0,036 mol) Triethylamin in 100 ml Tetrahydrofuran gelöst. Bei 0°C gibt

./.

0226132

man 10,3 g (0,05 mol) Dicyclohexylcarbodiimid (DCC) gelöst in 30 ml THF zu und rührt 24 h bei 0°C. Überschüssiges DCC zersetzt man mit 2 ml Eisessig, filtriert von Dicyclohexylharnstoff ab und entfernt das Lösungsmittel im Vakuum. Man nimmt in Methylenchlorid auf, wäscht die organ. Phase mit 1 N HCl, $NaHCO_3$-Lösung und Wasser, trocknet mit $Na_2SO_4$ und engt ein. Es hinterbleiben 17,5 g roher N-Cbo-L-Phenylalanyl-DL-phosphinothricindimethylester, der nur Abspaltung der Cbo-Gruppe in 100 ml Methanol gelöst wird. Man gibt 3,5 g konz. HCl und 4 g Palladium auf Aktivkohle (5%) zu und leitet bei 40°C $H_2$ ein bis die $CO_2$-Entwicklung beendet ist. Abfiltrieren des Katalysators und Eindampfen liefert 12,4 g rohes N-L-Phenylalanyl-DL-phosphinothricin dimethylesterhydrochlorid, das zur Verseifung der Estergruppen in 100 ml konz. HCl 5 h auf 60°C erwärmt wird. Nach Eindampfen und Trocknen erhält man 7,7 g rohes N-L-Phenylalanyl-DL-phosphinothricinhydrochlorid, das man zur Freisetzung in 100 ml abs. Ethanol löst. Nach Zugabe von 10 ml Propylenoxid rührt man 10 h bei Raumtemperatur, filtriert ab und trocknet. Man erhält 4,1 g (Ausbeute über alle Stufen 35%) N-L-Phenylalanyl-DL-phosphinothricin vom Schmp. 121-123°C (Zersetzung).

Die nachfolgenden in Tabelle 1 aufgeführten Verbindungen der Formel I können in analoger Weise erhalten werden.

./.

0226132

TABELLE 1 *

H-Aminosäure I-Aminosäure II-OH

| Bsp.Nr. | Aminosäure I | Aminosäure II | | Fp [°C] |
|---|---|---|---|---|
| 3 | Gly | L-Ptc | | |
| 4 | Gly | DL-Ptc | | |
| 5 | Gly | DL-Ptc | HCl-Salz | |
| 6 | Gly | DL-Ptc | Na-Salz | |
| 7 | Gly | L-Ptc | HCl-Salz | |
| 8 | Gly | L-Ptc | Na-Salz | |
| 9 | L-Ala | L-Ptc | | |
| 10 | L-Ala | DL-Ptc | | 182 (Zers.) |
| 11 | DL-Ala | L-Ptc | | |
| 12 | DL-Ala | DL-Ptc | | |
| 13 | L-Ala | L-Ptc | HCl-Salz | |
| 14 | L-Ala | L-Ptc | Na-Salz | |
| 15 | L-Ala | DL-Ptc | HCl-Salz | |
| 16 | L-Ala | DL-Ptc | Na-Salz | |
| 17 | ß-Ala | L-Ptc | | |
| 18 | ß-Ala | DL-Ptc | | |
| 19 | L-Val | L-Ptc | | |
| 20 | L-Val | DL-Ptc | | |
| 21 | DL-Val | L-Ptc | | |
| 22 | DL-Val | DL-Ptc | | |
| 23 | L-Leu | L-Ptc | | |

./.

0226132

Fortsetzung TABELLE 1

| Bsp.Nr. | Aminosäure I | Aminosäure II | | Fp [°C] |
|---|---|---|---|---|
| 24 | L-Leu | DL-Ptc | | 125-130 (Zers.) |
| 25 | DL-Leu | L-Ptc | | |
| 26 | DL-Leu | DL-Ptc | | |
| 27 | L-Ile | L-Ptc | | |
| 28 | L-Ile | DL-Ptc | | |
| 29 | DL-Ile | L-Ptc | | |
| 30 | DL-Ile | DL-Ptc | | |
| 31 | L-Phg | L-Ptc | | |
| 32 | L-Phg | DL-Ptc | | |
| 33 | DL-Phg | L-Ptc | | |
| 34 | DL-Phg | DL-Ptc | | 155 (Zers.) |
| 35 | D-Phe | DL-Ptc | | 135 (Zers.) |
| 36 | L-Phe | DL-Ptc | HCl-Salz | |
| 37 | DL-Phe | L-Ptc | | |
| 38 | DL-Phe | DL-Ptc | | 132-3 (Zers.) |
| 39 | L-Ser | L-Ptc | | |
| 40 | L-Ser | DL-Ptc | | |
| 41 | DL-Ser | L-Ptc | | |
| 42 | DL-Ser | DL-Ptc | | |
| 43 | L-Thr | L-Ptc | | |

./.

Fortsetzung TABELLE 1

| Bsp.Nr. | Aminosäure I | Aminosäure II | Fp [°C] |
|---|---|---|---|
| 44 | L-Thr | DL-Ptc | |
| 45 | DL-Thr | L-Ptc | |
| 46 | DL-Thr | DL-Ptc | |
| 47 | L-Met | L-Ptc | |
| 48 | L-Met | DL-Ptc | |
| 49 | DL-Met | L-Ptc | |
| 50 | DL-Met | DL-Ptc | |
| 51 | L-Tyr | L-Ptc | |
| 52 | L-Tyr | DL-Ptc | |
| 53 | DL-Tyr | L-Ptc | |
| 54 | DL-Tyr | DL-Ptc | |
| 55 | L-Glu | L-Ptc | |
| 56 | L-Glu | DL-Ptc | |
| 57 | DL-Glu | L-Ptc | |
| 58 | DL-Glu | DL-Ptc | |
| 59 | L-Gln | L-Ptc | |
| 60 | L-Gln | DL-Ptc | |
| 61 | DL-Gln | L-Ptc | |
| 62 | DL-Gln | DL-Ptc | |
| 63 | L-Asp | L-Ptc | |
| 64 | L-Asp | DL-Ptc | |

./.

Fortsetzung TABELLE 1

| Bsp.Nr. | Aminosäure I | Aminosäure II | | Fp [°C] |
|---|---|---|---|---|
| 65 | DL-Asp | L-Ptc | | |
| 66 | DL-Asp | DL-Ptc | | |
| 67 | L-Asn | L-Ptc | | |
| 68 | L-Asn | DL-Ptc | | |
| 69 | DL-Asn | L-Ptc | | |
| 70 | DL-Asn | DL-Ptc | | |
| 71 | L-Lys | L-Ptc | | |
| 72 | L-Lys | DL-Ptc | HCl-Salz | Glas |
| 73 | DL-Lys | L-Ptc | | |
| 74 | L-Lys | DL-Ptc | | |
| 75 | L-Nle | L-Ptc | | |
| 76 | L-Nle | DL-Ptc | | |
| 77 | DL-Nle | L-Ptc | | |
| 78 | DL-Nle | DL-Ptc | | |
| 79 | L-Nva | L-Ptc | | |
| 80 | L-Nva | DL-Ptc | | |
| 81 | DL-Nva | L-Ptc | | |
| 82 | DL-Nva | DL-Ptc | | 155 (Zers.) |
| 83 | L-Cys | L-Ptc | | |
| 84 | L-Cys | DL-Ptc | | |
| 85 | DL-Cys | L-Ptc | | |
| 86 | DL-Cys | DL-Ptc | | |

./.

Fortsetzung TABELLE 1

| Bsp.Nr. | Aminosäure I | Aminosäure II | Fp [°C] |
|---|---|---|---|
| 87 | L-Abu | L-Ptc | |
| 88 | L-Abu | DL-Ptc | |
| 89 | DL-Abu | L-Ptc | |
| 90 | DL-Abu | DL-Ptc | |
| 91 | γ-Abu | L-Ptc | |
| 92 | γ-Abu | DL-Ptc | |
| 93 | DL-Phg(4Cl) | L-Ptc | |
| 94 | DL-Phg(4Cl) | DL-Ptc | |
| 95 | DL-Phg (2Cl 4$CF_3$) | L-Ptc | |
| 96 | DL-Phg (2Cl 4$CF_3$) | DL-Ptc | |
| 97 | DL-Phe (4Cl) | L-Ptc | |
| 98 | DL-Phe (4Cl) | DL-Ptc | |
| 99 | DL-Phe (4$NO_2$) | L-Ptc | |
| 100 | DL-Phe (4$NO_2$) | DL-Ptc | |
| 101 | DL-Val (αMe) | L-Ptc | |
| 102 | DL-Val (αMe) | DL-Ptc | |

* Die einzelnen Aminosäuren werden durch Abkürzungen bezeichnet, die in der Peptidchemie allgemein gebräuchlich sind (Houben-Weyl Bd. 15/1, S. 2 ff). Phosphinothricin erhält die Abkürzung "Ptc". In Großbuchstaben vor der jeweiligen Aminosäure wird die Stereochemie der vorhandenen Asymmetriezentren angegeben.

./.

B. Formulierungsbeispiele

1. Eine 20%ige wässrige Lösung wird erhalten aus:
   20 Gew.-% Wirkstoff
   10 Gew.-% Nonylphenol x 10 AeO
   20 Gew.-% Ethylenglykol
   50 Gew.-% Wasser

2. Ein in Wasser leicht dispergierbares, benetzbares Pulver wird erhalten, indem man
   25 Gew.-% Wirkstoff
   64 Gew.-% kaolinhaltiges Quarz als Inertstoff
   10 Gew.-% ligninsulfonsaures Kalium und
   1 Gew.-% oleylmethyltaurinsaures Natrium als Netz- und Dispergiermittel
   mischt und in einer Stiftmühle mahlt.

## C. Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0-5 ausgedrückt ist. Dabei bedeutet:

0 = ohne Wirkung bzw. Schaden
1 = 0 - 20% Wirkung bzw. Schaden
2 =20 - 40% Wirkung bzw. Schaden
3 =40 - 60% Wirkung bzw. Schaden
4 =60 - 80% Wirkung bzw. Schaden
5 =80 -100% Wirkung bzw. Schaden

### 1. Unkrautwirkung im Nachauflauf

Samen bzw. Rhizomstücke von mono- und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver, als Emulsionskonzentrate bzw. als wässrige Lösungen formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3-4 Wochen Standzeit der Versuchspflanzen im Gewächshaus unter optimalen Wachstumsbedingungen (Temperatur 23 plus/minus 1°C, relative Luftfeuchte 60-80%) die Wirkung der Präparate optisch im Vergleich zu unbehandelten Kontrollen bonitiert. Die erfindungsgemäßen

./.

Mittel weisen im Nachauflauf eine gute herbizide Wirksamkeit gegen ein breites Spektrum wirtschaftlich wichtiger Ungräser und Unkräuter auf.

TABELLE 2

Herbizide Wirkung der erfindungsgemäßen Verbindungen; Nachauflaufverfahren

| Produkt (Bsp.Nr.) | Dosis kg a.i./ha | herbizide Wirkung AVF | SAL | ECG | SIA | CRS | STM |
|---|---|---|---|---|---|---|---|
| 1 | 2,4 | 5 | 5 | 5 | 5 | 5 | 5 |
| | 0,6 | 4 | 4 | 5 | 5 | 5 | 4 |
| 2 | 2,4 | 5 | 5 | 5 | 5 | 5 | 5 |
| | 0,6 | 5 | 5 | 5 | 5 | 5 | 4 |
| 24 | 2,4 | - | - | 5 | 5 | 5 | 5 |
| | 0,6 | - | - | - | - | - | - |
| 72 | 2,4 | - | - | 5 | 5 | 5 | 5 |
| | 0,6 | - | - | - | - | - | - |
| 34 | 2,4 | 4 | 5 | 5 | 5 | 5 | 5 |
| | 0,6 | 3 | 4 | 5 | 5 | 5 | 4 |

Abkürzungen:

AVF = Avena fatua (Flughafer)
SAL = Setaria lutescens (Borstenhirse)
ECG = Echinochloa crus-galli (Hühnerhirse)
SIA = Sinapis alba (Weißer Senf)
CFS = Chrysanthemum segetum (Saatwucherblume)
STM = Stellaria media (Sternmiere)
a.i.= Aktivsubstanz

Patentansprüche:

1. Verbindungen der allgemeinen Formel I,

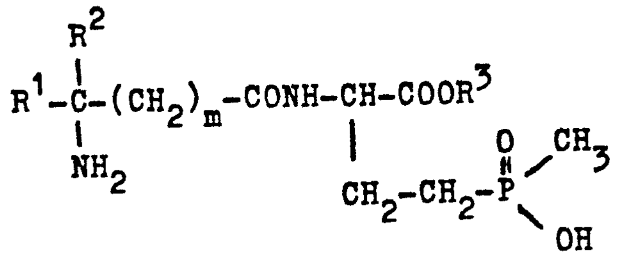

(I)

worin

$R^1$ und $R^2$ unabhängig voneinander Wasserstoff, $(C_1-C_{12})$-Alkyl, $(C_3-C_8)$Cycloalkyl, Phenyl, Benzyl oder Phenethyl, wobei die genannten Gruppen ihrerseits durch Halogen, $NH_2$, OH, SH, $NO_2$, $(C_1-C_4)$-Alkoxy, $(C_1-C_4)$Alkylthio, $CF_3$, $(C_1-C_4)$Alkoxycarbonyl, COOH oder $CONH_2$ substituiert sein können, oder

$R^1$ und $R^2$ gemeinsam eine Alkylenkette von 2 - 7 C-Atomen,

$R^3$ Wasserstoff oder $(C_1-C_{12})$-Alkyl und

m 0, 1, 2 oder 3

bedeuten, sowie deren Salze mit Basen und Säuren.

2. Verbindungen der Formel I, worin

$R^1$ und $R^2$ unabhängig voneinander Wasserstoff, $(C_1-C_8)$-Alkyl, Phenyl, Benzyl und Phenylethyl, wobei die genannten Gruppen ihrerseits durch $NH_2$, OH, SH, $(C_1-C_4)$Alkoxy, $(C_1-C_4)$Alkylthio, $(C_1-C_4)$-Alkoxycarbonyl, COOH oder $CONH_2$ substituiert sein können,

$R^1$ und $R^2$ zusammen mit dem sie verbindenden C-Atom ein Cyclopentyl- oder Cyclohexylrest,

$R^3$ Wasserstoff oder Methyl und m 0,1 oder 2 bedeuten, sowie deren Salze.

3. Verfahren zur Herstellung von Verbindungen der Formel I gemäß den Ansprüchen 1 oder 2, dadurch gekennzeichnet daß man

a) eine Verbindung der Formel II

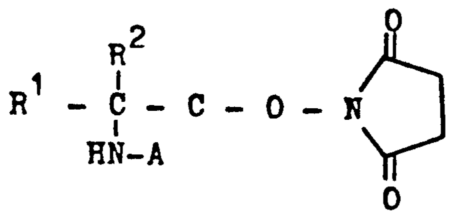

(II)

worin A eine Amino-Schutzgruppe bedeutet, mit Phosphinothricin in Gegenwart einer Hilfsbase oder

b) eine Verbindung der Formel III

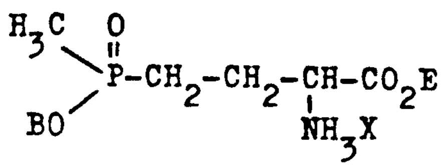

(III)

worin B und E Säureschutzgruppen sind und X das Anion-äquivalent einer starken Säure bedeutet, mit einer Verbindung der Formel IV

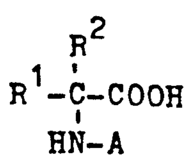

(IV)

in Gegenwart einer Hilfsbase und eines Kondensationsmittels

umsetzt und anschließend, soweit gewünscht, die vorhandenen Schutzgruppen in bekannter Weise abspaltet.

4. Mittel zur Unkrautbekämpfung, gekennzeichnet durch einen Gehalt an einer Verbindung der Formel I gemäß den Ansprüchen 1 oder 2.

5. Verwendung von Verbindungen der Formel I gemäß den Ansprüchen 1 oder 2 zur Bekämpfung unerwünschten Pflanzenwuchses.

6. Verfahren zur Bekämpfung unerwünschten Pflanzenwuchses, dadurch gekennzeichnet, daß man auf die zu bekämpfenden Pflanzen bzw. die zu behandelnde Fläche im Vorsaat-, Vorauflauf- oder Nachauflaufverfahren eine wirksame Menge einer Verbindung der Formel I gemäß den Ansprüchen 1 oder 2 aufbringt.

Patentansprüche Österreich:

1. Verfahren zur Herstellung von Verbindungen der Formel I,

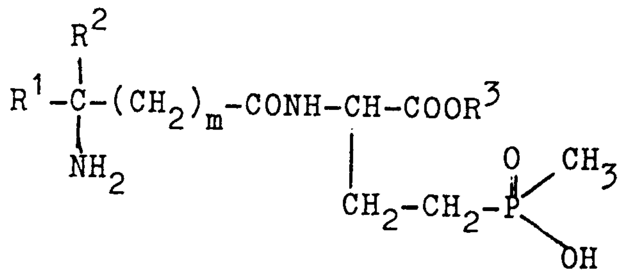

(I)

worin

$R^1$ und $R^2$ unabhängig voneinander Wasserstoff, $(C_1-C_{12})$-Alkyl, $(C_3-C_8)$Cycloalkyl, Phenyl, Benzyl oder Phenethyl, wobei die genannten Gruppen ihrerseits durch Halogen, $NH_2$, OH, SH, $NO_2$, $(C_1-C_4)$-Alkoxy, $(C_1-C_4)$Alkylthio, $CF_3$, $(C_1-C_4)$Alkoxycarbonyl, COOH oder $CONH_2$ substituiert sein können, oder

$R^1$ und $R^2$ gemeinsam eine Alkylenkette von 2 - 7 C-Atomen,

$R^3$ Wasserstoff oder $(C_1-C_{12})$-Alkyl und

m 0, 1, 2 oder 3

bedeuten, sowie von deren Salzen mit Basen und Säuren, dadurch gekennzeichnet, daß man

a) eine Verbindung der Formel II

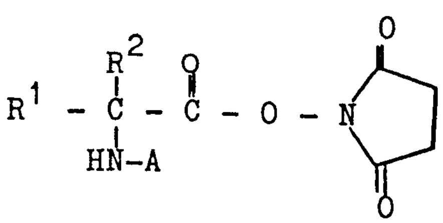

(II)

worin A eine Amino-Schutzgruppe bedeutet, mit Phosphinothricin in Gegenwart einer Hilfsbase oder

b) eine Verbindung der Formel III

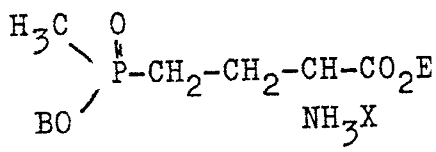

(III)

worin B und E Säureschutzgruppen sind und X das Anionäquivalent einer starken Säure bedeutet, mit einer Verbindung der Formel IV

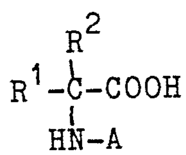

(IV)

in Gegenwart einer Hilfsbase und eines Kondensationsmittels umsetzt und anschließend, soweit gewünscht, die vorhandenen Schutzgruppen in bekannter Weise abspaltet.

2. Verfahren zur Herstellung von Verbindungen der Formel I worin

$R^1$ und $R^2$ unabhängig voneinander Wasserstoff, $(C_1-C_8)$-Alkyl, Phenyl, Benzyl und Phenylethyl, wobei die genannten Gruppen ihrerseits durch $NH_2$, OH, SH, $(C_1-C_4)$Alkoxy, $(C_1-C_4)$Alkylthio, $(C_1-C_4)$-Alkoxycarbonyl, COOH oder $CONH_2$ substituiert sein können,

$R^1$ und $R^2$ zusammen mit dem sie verbindenden C-Atom ein Cyclopentyl- oder Cyclohexylrest,

$R^3$ Wasserstoff oder Methyl und m 0,1 oder bedeuten, sowie von deren Salzen,

dadurch gekennzeichnet, daß man

a) eine Verbindung der Formel II

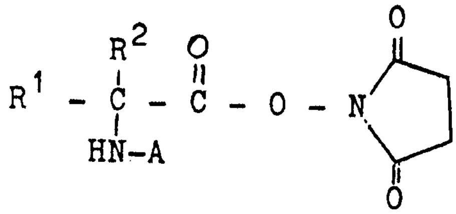

(II)

worin A eine Amino-Schutzgruppe bedeutet, mit Phosphinothricin in Gegenwart einer Hilfsbase oder

b) eine Verbindung der Formel III

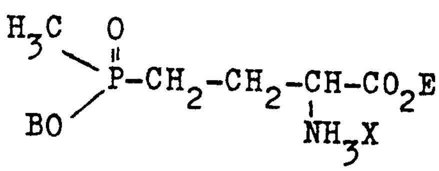

(III)

worin B und E Säureschutzgruppen sind und X das Anionäquivalent einer starken Säure bedeutet, mit einer Verbindung der Formel IV

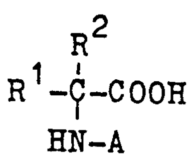

(IV)

in Gegenwart einer Hilfsbase und eines Kondensationsmittel umsetzt und anschließend, soweit gewünscht, die vorhandenen Schutzgruppen in bekannter Weise abspaltet.

3. Mittel zur Unkrautbekämpfung, gekennzeichnet durch einen Gehalt an einer Verbindung der Formel I gemäß den Ansprüchen 1 oder 2.

4. Verwendung von Verbindungen der Formel I gemäß den Ansprüchen 1 oder 2 zur Bekämpfung unerwünschten Pflanzenwuchses.

5. Verfahren zur Bekämpfung unerwünschten Pflanzenwuchses, dadurch gekennzeichnet, daß man auf die zu bekämpfenden Pflanzen bzw. die zu behandelnde Fläche im Vorsaat-, Vorauflauf- oder NAchauflaufverfahren eine wirksame Menge einer Verbindung der Formel I gemäß den Ansprüchen 1 oder 2 aufbringt.